# EUROPÄISCHE PATENTANMELDUNG

(11) **EP 3 885 001 A1**
(43) Veröffentlichungstag der Anmeldung: **29.09.2021**
(21) Anmeldenummer: 21163187.4
(22) Anmeldetag: 17.03.2021
(51) Int. Cl.: A61N 5/10, A61B 90/96, A61B 90/98

(54) **BRACHYTHERAPIESYSTEM**

(30) Priorität: 26.03.2020 DE 102020108352
(71) Anmelder: Carl Zeiss Meditec AG, 07745 Jena (DE)
(72) Erfinder: Symalla, Michael, 73430 Aalen (DE); Röder, Norman, 99441 Döbritschen (DE)
(74) Vertreter: Diehl & Partner

(57) **Zusammenfassung**

Die Erfindung betrifft ein Brachytherapiesystem. Das Brachytherapiesystem umfasst eine Strahlungserzeugungsvorrichtung (3) zum Erzeugen von Röntgenstrahlung, eine Steuerung (27) zum Steuern der Strahlungserzeugungsvorrichtung (3), einen Applikator (15), welcher so ausgebildet ist, dass er auf die Strahlungserzeugungsvorrichtung (3) aufgesetzt werden kann, und ein Informationsübertragungssystem (29B), welches dazu konfiguriert ist, eine in oder an dem Applikator (15) verkörperte Information zu erfassen, wenn der Applikator (15) auf die Strahlungserzeugungsvorrichtung (3) aufgesetzt ist, und die erfasste Information an die Steuerung (27) zu übertragen. Die Steuerung (27) ist dazu konfiguriert, die Erzeugung von Röntgenstrahlung und das Ausgeben von Warnungen in Abhängigkeit der empfangenen Information zu steuern.

## Beschreibung

Die vorliegende Erfindung liegt in dem Bereich der medizinischen Strahlentherapie und betrifft ein Brachytherapiesystem.

Die Brachytherapie ist ein Verfahren zur Therapierung von Gewebe im Inneren eines menschlichen oder tierischen Körpers mittels Röntgenstrahlung. Die Brachytherapie wird insbesondere im Anschluss an eine Resektion von Tumorgewebe durchgeführt. Die Brachytherapie wird mit einem Brachytherapiesystem durchgeführt.

Herkömmliche Brachytherapiesysteme umfassen eine Strahlungserzeugungsvorrichtung und einen Applikator.

Die Strahlungserzeugungsvorrichtung dient der Erzeugung von Röntgenstrahlung. Eine herkömmliche Strahlungserzeugungsvorrichtung umfasst ein Partikelstrahlsystem, welches einen hochenergetischen Partikelstrahl erzeugen kann. Der Partikelstrahl wird durch ein einige Zentimeter langes Rohr der Strahlungserzeugungsvorrichtung auf ein Röntgen-Material gerichtet, welches am Ende des Rohres angeordnet ist. Durch Wechselwirkung des Partikelstrahls mit dem Röntgen-Material erzeugt dieses Röntgenstrahlung, welche zur Therapierung von Gewebe vorgesehen ist.

Damit die von dem Röntgen-Material an der Spitze des Rohres erzeugte Röntgenstrahlung im Inneren eines Körpers angewendet werden kann, wird das Rohr in den Körper eingeführt. Hierzu ist das Rohr von einem auf die Strahlungserzeugungsvorrichtung aufsetzbaren Applikator umgeben, welcher einerseits eine sterile Barriere darstellt und andererseits das Rohr des Strahlentherapiegeräts schützt.

Herkömmlicherweise wird vor der Bestrahlung ein Applikator durch eine Person ausgewählt und auf die Strahlungserzeugungsvorrichtung aufgesetzt. Die Auswahl hat in der Regel nach einem Bestrahlungsplan zu erfolgen, welcher von einem Arzt festgelegt wird und den für die Bestrahlung zu verwenden Applikator angibt. Beispielweiseweise gibt der Bestrahlungsplan die für die Bestrahlung zu verwendende Größe und den Typ des Applikators und die Bestrahlungsdauer und die Bestrahlungsstärke an.

Ein Problem an diesem herkömmlichen Verfahren ist, dass die Person einen Applikator auswählen und für die Bestrahlung verwenden kann, der nicht dem Bestrahlungsplan entspricht. Dies kann zu einer fehlerhaften Therapie führen. Außerdem existiert keine zuverlässige Möglichkeit zur Protokollierung der Nutzung eines Applikators.

Die vorangehend genannten Probleme werden durch ein Brachytherapiesystem gemäß Anspruch 1 gelöst. Die abhängigen Ansprüche umfassen vorteilhafte und spezielle Weiterbildungen des Brachytherapiesystems.

Ausführungsformen der Erfindung werden nachfolgend anhand von Figuren näher erläutert. Hierbei zeigt:
- Figur 1: eine schematische Darstellung eines Brachytherapiesystems,
- Figur 2: eine schematische Darstellung eines Informationsübertragungssystems gemäß einer Ausführungsform,
- Figur 3: eine schematische Darstellung eines Applikators in der Ebene A-A,
- Figur 4: eine schematische Darstellung eines Informationsübertragungssystems gemäß einer weiteren Ausführungsform,
- Figur 5: eine schematische Darstellung eines Informationsübertragungssystems gemäß einer weiteren Ausführungsform,
- Figur 6: eine schematische Darstellung eines Informationsübertragungssystems gemäß einer weiteren Ausführungsform,
- Figur 7: ein Verfahren zur Anwendung des Brachytherapiesystems, und
- Figur 8: ein weiteres Verfahren zur Anwendung des Brachytherapiesystems.

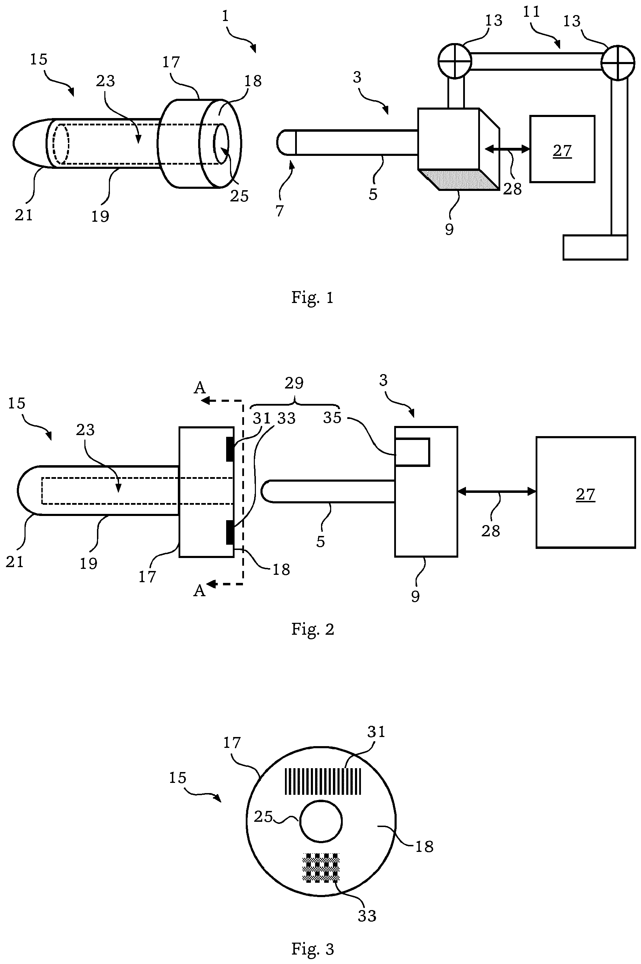

Figur 1 zeigt eine schematische Darstellung eines Brachytherapiesystems 1. Das Brachytherapiesystem 1 umfasst eine Strahlungserzeugungsvorrichtung 3, welche dazu konfiguriert ist, Röntgenstrahlung zu erzeugen. Die Strahlungserzeugungsvorrichtung 3 umfasst ein Rohr 5, an dessen einem Ende Röntgen-Material 7 angeordnet ist. Die Strahlungserzeugungsvorrichtung 3 umfasst an dem anderen Ende des Rohres 5 ein Gehäuse 9, in welchem eine in den Figuren nicht dargestellte Teilchenstrahlvorrichtung enthalten ist, welche dazu konfiguriert ist, einen Teilchenstrahl zu erzeugen und durch das Rohr 5 auf das Röntgen-Material 7 zu richten. Durch Wechselwirkung des Teilchenstrahls mit dem Röntgen-Material 7 wird Röntgenstrahlung erzeugt, die zur Therapierung verwendet werden kann.

Die Strahlungserzeugungsvorrichtung 3 ist durch ein bewegliches Stativ 11 gehalten. In dem in Figur 1 gezeigten Beispiel umfasst das Stativ 11 mehrere Gelenke 13, welche es erlauben, die Strahlungserzeugungsvorrichtung 3 variabel zu positionieren und zu orientieren. Das Stativ 11 kann automatisiert sein. Beispielsweise umfasst das Stativ 11 einen oder mehrere Aktuatoren, welche die Gelenke 13 verstellen können. Das Stativ 11 kann von einer Steuerung 27 des Brachytherapiesystems 1 gesteuert werden.

Das Brachytherapiesystem 1 umfasst ferner einen Applikator 15, welcher so ausgebildet ist, dass er auf die Strahlungserzeugungsvorrichtung 3 aufgesetzt werden kann. Zur Therapierung von Gewebe wird der Applikator 15 in das Gewebe eingeführt. Je nach gewünschter Bestrahlung kann der Applikator 15 unterschiedliche Formen und Größen haben. In der Praxis wird dem Bediener des Brachytherapiesystems 1 eine Vielzahl von Applikatoren unterschiedlicher Größe und Gestalt bereitgestellt, wobei jeder der Applikatoren auf die Strahlungserzeugungsvorrichtung 3 aufgesetzt werden kann.

Der Applikator 15 umfasst einen Anschlussabschnitt 17, einen rohrförmigen Abschnitt 19 und eine Spitze 21.

Der Anschlussabschnitt 17 und der rohrförmige Abschnitt 19 weisen einen zusammenhängenden Hohlraum 23 auf, welcher in eine Öffnung 25 in dem Anschlussabschnitt 17 mündet. Die Öffnung 25 und der Hohlraum 23 sind so ausgebildet, dass das Rohr 5 der Strahlungserzeugungsvorrichtung 3 durch die Öffnung 25 in den Hohlraum 23 des Applikators 15 eingeführt werden kann. Der Hohlraum 23 kann sich bis in die Spitze 21 des Applikators 15 erstrecken. Der Applikator 15 wird auf die Strahlungserzeugungsvorrichtung 3 aufgesetzt, indem das Rohr 5 der Strahlungserzeugungsvorrichtung 3 durch die Öffnung 25 des Applikators 15 in den Hohlraum 23 des Applikators 15 geführt wird.

Die Spitze 21 kann, wie in Figur 1 gezeigt, abgerundet sein. Alternativ kann die Spitze eine andere Gestalt aufweisen, beispielsweise eine kugelförmige Gestalt oder dergleichen.

An dem Gehäuse 9 der Strahlungserzeugungsvorrichtung 3 und dem Anschlussabschnitt 17 des Applikators 15 kann eine in den Figuren nicht dargestellte Haltungsvorrichtung angeordnet sein, welche dazu konfiguriert ist, eine lösbare Verbindung zwischen dem Gehäuse 9 der Strahlungserzeugungsvorrichtung 3 und dem Anschlussabschnitt 17 des Applikators 15 bereitzustellen. Hierdurch kann der Applikator 15 an der Strahlungserzeugungsvorrichtung 3 gehaltert werden. Außerdem kann der Applikator 15 von der Strahlungserzeugungsvorrichtung 3 schadlos gelöst und entfernt werden. Beispiele für eine solche Haltungsvorrichtung sind ein Bajonettverschluss, ein Magnetverschluss und dergleichen.

Das Brachytherapiesystem 1 umfasst ferner eine Steuerung 27, welche dazu konfiguriert ist, die Strahlungserzeugungsvorrichtung 3 zu steuern. Beispielsweise steuert die Steuerung 27 die Erzeugung von Röntgenstrahlung durch die Strahlungserzeugungsvorrichtung 3. Die Steuerung 27 kann ferner das Stativ 11 steuern. Weitere Funktionen der Steuerung 27 werden später mit Bezug zu den weiteren Figuren erläutert.

Das Brachytherapiesystem 1 umfasst ferner ein Informationsübertragungssystem. Ein beispielhaftes Informationsübertragungssystem 29 und dessen Verwendung wird mit Bezug zu den Figuren 2 und 3 erläutert.

Figur 2 zeigt in schematischer Darstellung den Applikator 15 und die Strahlungserzeugungsvorrichtung 3 von der Seite. Figur 3 zeigt eine Oberfläche 18 des Anschlussabschnitts 17 des Applikators 15, die dem Gehäuse 9 der Strahlungserzeugungsvorrichtung 3 zugewandt ist, wenn der Applikator 15 auf die Strahlungserzeugungsvorrichtung 3 aufgesetzt ist. Die Perspektive der Figur 3 ist in Figur 2 mit der Schnittkennzeichnung A-A dargestellt.

Das Informationsübertragungssystem 29 ist dazu konfiguriert, eine in oder an dem Applikator 15 verkörperte Information zu erfassen, wenn der Applikator 15 auf die Strahlungserzeugungsvorrichtung 3 aufgesetzt ist. Ferner ist das Informationsübertragungssystem 29 dazu konfiguriert, die erfasste Information an die Steuerung 27 zu übertragen, wenn der Applikator 15 auf die Strahlungserzeugungsvorrichtung 3 aufgesetzt ist.

Das Informationsübertragungssystem 29 kann so konfiguriert sein, dass die Information nicht erfasst werden kann, wenn der Applikator 15 nicht auf die Strahlungserzeugungsvorrichtung 3 aufgesetzt ist. Dementsprechend kann die Information nur dann erfasst werden, wenn der Applikator 15 auf die Strahlungserzeugungsvorrichtung 3 aufgesetzt ist. Zudem kann das Informationsübertragungssystem 29 so konfiguriert sein, dass die erfasste Information nicht an die Steuerung 27 übertragen werden kann, wenn der Applikator 15 nicht auf die Strahlungserzeugungsvorrichtung 3 aufgesetzt ist. Dementsprechend kann die erfasste Information nur dann an die Steuerung 27 übertragen werden, wenn der Applikator 15 auf die Strahlungserzeugungsvorrichtung 3 aufgesetzt ist.

In dem Beispiel der Figuren 2 und 3 umfasst das Informationsübertragungssystem 29 eine optische Kennzeichnung 31, 33 und ein Lesegerät 35. Die optische Kennzeichnung kann beispielsweise ein Strichcode 31, ein QR-Code 33 oder dergleichen sein. Die optische Kennzeichnung 31, 33 ist beispielsweise an der Oberfläche 18 des Applikators 15 angeordnet, die dem Gehäuse 9 der Strahlungserzeugungsvorrichtung 3 zugewandt ist, wenn der Applikator 15 auf die Strahlungserzeugungsvorrichtung 3 aufgesetzt ist. Das Lesegerät 35 ist dazu konfiguriert, die optische Kennzeichnung 31, 33 zu lesen. Die durch die optische Kennzeichnung 31, 33 verkörperte Information an dem Applikator 15 kann somit durch das Lesegerät 35 gelesen werden. Das Lesegerät 35 kann die durch Lesen der optischen Kennzeichnung 31, 33 erfasste Information an die Steuerung 27 übertragen. Eine Kommunikationsverbindung 28 zwischen dem Lesegerät 35 bzw. der Strahlungserzeugungsvorrichtung 3 und der Steuerung 27 zur Übertragung der Information ist schematisch durch einen Doppelpfeil dargestellt.

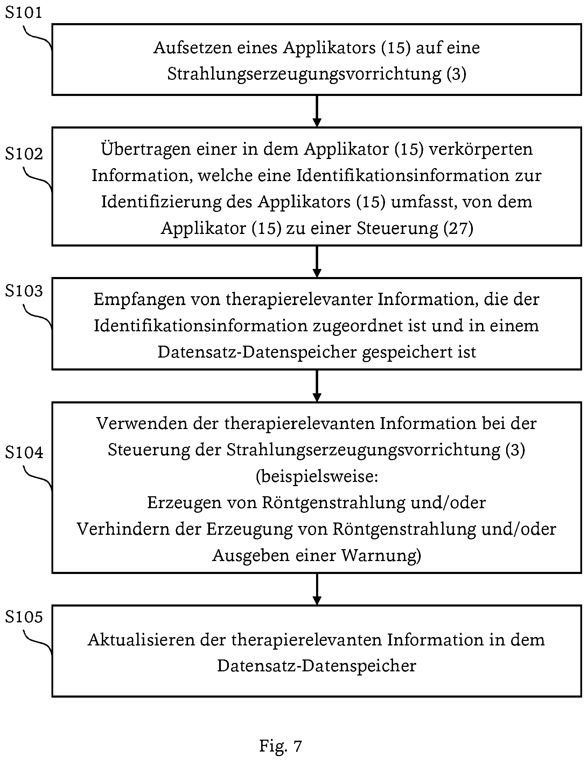

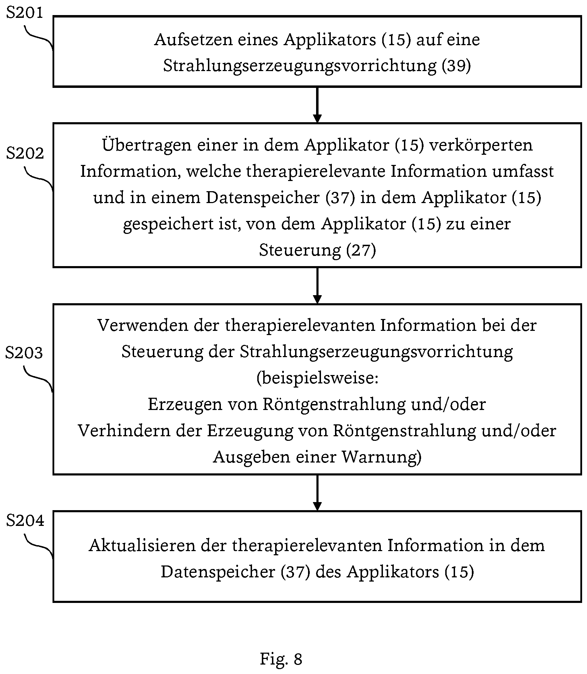

Die Steuerung 27 empfängt die Information, die durch das Informationsübertragungssystem 29 erfasst und gesendet wurde, und verwendet diese Information im Zusammenhang mit der Erzeugung von Röntgenstrahlung durch die Strahlungserzeugungsvorrichtung 3. Mit anderen Worten steuert die Steuerung 27 die Erzeugung von Röntgenstrahlung durch die Strahlungserzeugungsvorrichtung 3 in Abhängigkeit der erfassten und empfangenen Information. Weitere Details über die Bedeutung der Information sowie deren Verwendung bei der Erzeugung der Röntgenstrahlung werden später mit Bezug zu den Figuren 7 und 8 erläutert.

In dem in Figur 2 gezeigten Beispiel ist das Lesegerät 35 in die Strahlungserzeugungsvorrichtung 3 integriert. Das Lesegerät 35 kann jedoch auch separat von der Strahlungserzeugungsvorrichtung 3 bereitgestellt sein. Wesentlich ist jedoch, dass die in oder an dem Applikator 15 verkörperte Information in Form der optischen Kennzeichnung 31, 33 (nur) erfasst werden kann, wenn der Applikator 15 auf die Strahlungserzeugungsvorrichtung 3 aufgesetzt ist.

Dadurch, dass die in oder an dem Applikator 15 verkörperte Information (nur) dann erfasst werden kann, wenn der Applikator 15 auf die Strahlungserzeugungsvorrichtung 3 aufgesetzt ist, wird sichergestellt, dass die Information, die der Steuerung durch das Informationsübertragungssystem 29 bereitgestellt wird, tatsächlich von dem Applikator 15 stammt, der auf die Strahlungserzeugungsvorrichtung 3 aufgesetzt ist. Somit kann die Steuerung 27 für die Steuerung der Strahlungserzeugungsvorrichtung 3 die Information verwenden, die von dem aufgesetzten Applikator 15 stammt. Dementsprechend wird eine Verwechslungsgefahr hinsichtlich des für die Bestrahlung verwendeten Applikators 15 eliminiert, da die Information nicht etwa durch eine Person erfasst werden muss, sondern durch das Informationsübertragungssystem 29 am Applikator 15 erfasst und an die Steuerung 27 übertragen wird.

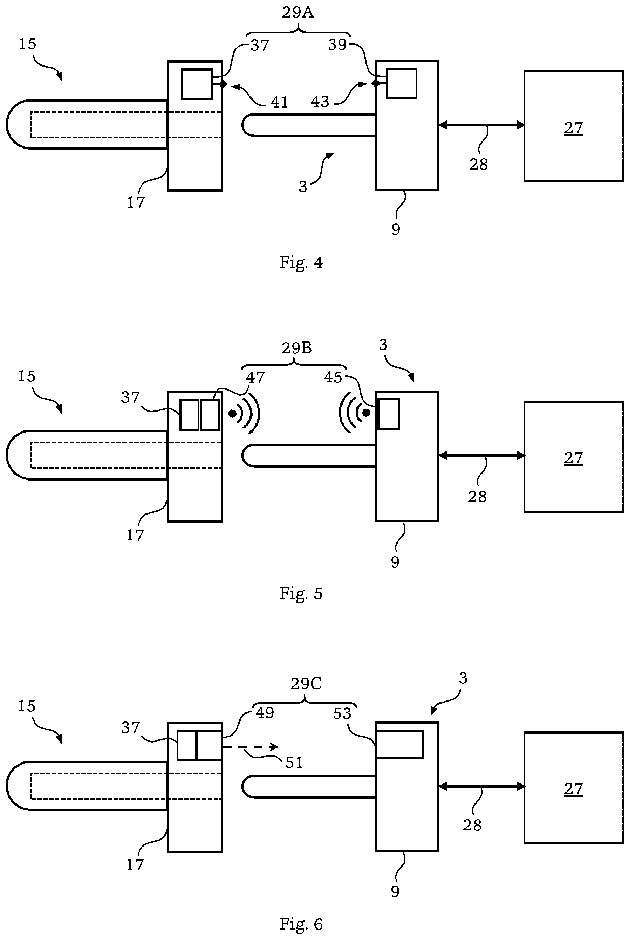

Mit Bezug zu den Figuren 4 bis 6 werden beispielhafte alternative Informationsübertragungssysteme erläutert.

Figur 4 zeigt eine schematische Darstellung eines Informationsübertragungssystems 29A gemäß einer weiteren Ausführungsform. In Figur 4 sind der Applikator 15 und die Strahlungserzeugungsvorrichtung 3 von der Seite gezeigt. Das Informationsübertragungssystem 29A umfasst einen Datenspeicher 37, welcher in dem Applikator 15 angeordnet ist. Die in dem Applikator 15 verkörperte Information wird in dieser Ausführungsform durch ein Signal realisiert, welches in dem Datenspeicher 37 gespeichert ist.

Das Informationsübertragungssystem 29A umfasst ferner eine Empfangseinheit 39. Die Empfangseinheit 39 ist dazu konfiguriert, die Information aus dem Datenspeicher 37 zu empfangen, wenn der Applikator 15 auf die Strahlungserzeugungsvorrichtung 3 aufgesetzt ist. Wie in Figur 4 gezeigt, kann die Empfangseinheit 39 in der Strahlungserzeugungsvorrichtung 3 integriert sein. Die Empfangseinheit 39 kann jedoch auch außerhalb der Strahlungserzeugungsvorrichtung 3 angeordnet sein, solange die Empfangseinheit 39 die in dem Datenspeicher 37 realisierte Information (nur dann) empfangen kann, wenn der Applikator 15 auf die Strahlungserzeugungsvorrichtung 3 aufgesetzt ist. Wenn die Empfangseinheit 39 die Information empfangen hat, überträgt die Empfangseinheit 39 die Information an die Steuerung 27.

In dem in Figur 4 gezeigten Beispiel weist der Applikator 15 an seiner Oberfläche wenigstens einen elektrischen Kontakt 41 auf, welcher eine Kommunikationsverbindung zu dem Datenspeicher 37 bereitstellt. Der elektrische Kontakt 41 befindet sich auf der Oberfläche 18 des Anschlussabschnitts 17 des Applikators 15, welche dem Gehäuse 9 der Strahlungserzeugungsvorrichtung 3 zugewandt ist, wenn der Applikator 15 auf die Strahlungserzeugungsvorrichtung 3 aufgesetzt ist.

Das Gehäuse 9 der Strahlungserzeugungsvorrichtung 3 umfasst an seiner Oberfläche wenigstens einen elektrischen Kontakt 43, der zu dem wenigstens einen elektrischen Kontakt 41 des Applikators 15 passt. Der wenigstens eine elektrische Kontakt 43 stellt eine Kommunikationsverbindung zu der Empfangseinheit 39 bereit.

Wenn der Applikator 15 auf die Strahlungserzeugungsvorrichtung 3 aufgesetzt ist, sind der wenigstens eine elektrische Kontakt 41 und der wenigstens eine elektrische Kontakt 43 miteinander elektrisch verbunden, wodurch eine drahtgebundene Kommunikationsverbindung zwischen dem Datenspeicher 37 und der Empfangseinheit 39 hergestellt ist. Somit kann die Empfangseinheit 39 die Information aus dem Datenspeicher 37 empfangen, wenn der Applikator 15 auf die Strahlungserzeugungsvorrichtung 3 aufgesetzt ist. Wenn der Applikator 15 nicht auf die Strahlungserzeugungsvorrichtung 3 aufgesetzt ist, ist die Kommunikationsverbindung unterbrochen und die Empfangseinheit 39 kann die Information nicht empfangen. Somit kann die Empfangseinheit 39 die durch das Signal in dem Datenspeicher 37 realisierte Information nur dann auslesen, wenn der Applikator 15 auf die Strahlungserzeugungsvorrichtung 3 aufgesetzt ist.

Die Empfangseinheit 39 überträgt die empfangene Information an die Steuerung 27. Somit kann die Steuerung 27 die empfangene Information zur Steuerung der Strahlungserzeugungsvorrichtung 3 verwenden.

Figur 5 zeigt eine schematische Darstellung eines Informationsübertragungssystems 29B gemäß einer weiteren Ausführungsform. Das Informationsübertragungssystem 29B ist dazu konfiguriert, die in Form des Signals in dem Datenspeicher 37 realisierte Information drahtlos an eine Empfangseinheit 45 zu übertragen. Hierzu umfasst das Informationsübertragungssystem 29B ein in oder an dem Applikator 15 angeordnetes Kommunikationsmodul 47. Das Kommunikationsmodul 47 ist dazu konfiguriert, die Information aus dem Datenspeicher 37 zu lesen und drahtlos an die Empfangseinheit 45 zu übertragen. Dementsprechend ist das Kommunikationsmodul 47 dazu konfiguriert, die Information drahtlos auszusenden; und die Empfangseinheit 45 ist dazu konfiguriert, die von dem Kommunikationsmodul 47 gesendete Information zu empfangen.

In dem in Figur 5 gezeigten Beispiel ist die Empfangseinheit 45 in der Strahlungserzeugungsvorrichtung 3 integriert. Die Empfangseinheit 45 kann jedoch auch außerhalb der Strahlungserzeugungsvorrichtung 3 angeordnet sein.

Das Kommunikationsmodul 47 und die Empfangseinheit 45 können so konfiguriert sein, dass die drahtlose Kommunikation nur über eine kurze Entfernung funktioniert. Hierdurch kann sichergestellt werden, dass die Übertragung der Information von dem Applikator 15 zu der Empfangseinheit 45 nur dann erfolgen werden kann, wenn der Applikator 15 auf die Strahlungserzeugungsvorrichtung 3 aufgesetzt ist.

Wenn die Empfangseinheit 45 die Information empfangen hat, überträgt die Empfangseinheit 45 die Information an die Steuerung 27. Die Steuerung 27 verwendet die empfangene Information zur Steuerung der Strahlungserzeugungsvorrichtung 3.

Das Kommunikationsmodul 47 kann beispielsweise ein RFID (radio frequency identification)-Tag sein. Das RFID-Tag kann an der Oberfläche des Applikators 15 oder im Inneren des Applikators 15 angeordnet sein.

Figur 6 zeigt eine schematische Darstellung eines Informationsübertragungssystems 29C gemäß einer weiteren Ausführungsform. In dem in Figur 6 gezeigten Beispiel umfasst das Informationsübertragungssystem 29C ein optisches Informationsübertragungssystem. Das optische Informationsübertragungssystem ist dazu konfiguriert, ein Lichtsignal 51 zur Übertragung der in dem Datenspeicher 37 gespeicherten Information zu verwenden. Beispielsweise umfasst das Informationsübertragungssystem 29C eine steuerbare Lichtquelle 49, welche in dem Applikator 15 angeordnet ist und mit dem Datenspeicher 37 verbunden ist. Die steuerbare Lichtquelle 49 ist dazu konfiguriert, ein Lichtsignal 51 auszusenden, welches die in dem Datenspeicher 37 realisierte Information trägt. Das Informationsübertragungssystem 29C umfasst ferner eine Empfangseinheit 53, welche dazu konfiguriert ist, das Lichtsignal 51 (nur dann) zu empfangen, wenn der Applikator 15 auf die Strahlungserzeugungsvorrichtung 3 aufgesetzt ist. In dem in Figur 6 gezeigten Beispiel ist die Empfangseinheit 53 in der Strahlungserzeugungsvorrichtung 3 integriert. Die Empfangseinheit 53 kann jedoch auch außerhalb der Strahlungserzeugungsvorrichtung 3 angeordnet sein, solange die Empfangseinheit 53 das Lichtsignal 51 empfangen kann, wenn der Applikator 15 auf die Strahlungserzeugungsvorrichtung 3 aufgesetzt ist.

Die Empfangseinheit 53 überträgt die empfangene Information an die Steuerung 27. Die Steuerung 27 verwendet die empfangene Information zur Steuerung der Strahlungserzeugungsvorrichtung 3.

Mit Bezug zu den Figuren 2 bis 6 wurden diverse Informationsübertragungssysteme beschrieben, welche dazu konfiguriert sind, die in oder an dem Applikator 15 verkörperte Information zu erfassen und an die Steuerung 27 zu übertragen, wenn der Applikator 15 auf die Strahlungserzeugungsvorrichtung 3 aufgesetzt ist. Nachfolgend werden mit Bezug zu den Figuren 7 und 8 unterschiedliche Ausführungsformen hinsichtlich der Bedeutung der Information und hinsichtlich der Verwendung der Information durch die Steuerung 27 erläutert.

Figur 7 zeigt ein Verfahren zur Anwendung des in Zusammenhang mit den Figuren 1 bis 6 erläuterten Brachytherapiesystems 1.

In einem Schritt S101 wird ein Applikator 15 auf eine Strahlungserzeugungsvorrichtung 3 aufgesetzt. Dies wird durchgeführt, indem das Rohr 5 der Strahlungserzeugungsvorrichtung 3 durch die Öffnung 25 in dem Anschlussabschnitt 17 des Applikators 15 eingeführt und weiter in den Hohlraum 23 des Applikators 15 eingeführt wird.

In Schritt S102, der auf Schritt S101 folgt, wird eine in oder an dem Applikator 15 verkörperte Information durch das Informationsübertragungssystem 29, 29A, 29B, 29C von dem Applikator 15 zu der Steuerung 27 übertragen, wobei die Information eine Identifikationsinformation ist, welche den Applikator 15 (eindeutig) identifiziert. Die Identifikationsinformation dient zur (eindeutigen) Identifizierung des Applikators 15. Die Identifikationsinformation kann beispielsweise eine Zahl sein, welche in Form einer optischen Kennzeichnung 31, 33 oder als Signal in dem Datenspeicher 37 realisiert ist.

Der Identifikationsinformation ist ein Datensatz zugeordnet. Der Datensatz umfasst therapierelevante Information. Damit ist einer Identifikationsinformation eine therapierelevante Information zugeordnet.

In Schritt S103, der auf Schritt S102 folgt, empfängt die Steuerung 27 den der Identifikationsinformation zugeordneten Datensatz aus einem Datensatz-Datenspeicher. Der Datensatz-Datenspeicher ist beispielsweise ein von dem Datenspeicher 37 verschiedener Speicher. Der Datensatz-Datenspeicher kann beispielsweise in Form einer Festplatte, einem allgemeinen Speichermedium, in einem verteilten Netzwerk und dergleichen implementiert sein. Zu jedem einer Vielzahl von Applikatoren kann in dem Datensatz-Datenspeicher ein Datensatz gespeichert sein, welcher therapierelevante Information über den jeweiligen Applikator enthält. Mittels der Identifikationsinformation ist eine eindeutige Zuordnung zwischen einem Applikator und einem Datensatz möglich, wobei der Datensatz sich allein auf einen einzigen Applikator bezieht.

Die therapierelevante Information umfasst Information, die den Applikator charakterisiert und für eine Bestrahlung relevant ist.

Beispielsweise umfasst die therapierelevante Information eine Typbezeichnung des Applikators 15. Applikatoren können unterschiedlich geformt sein. Den unterschiedlichen Formen kann jeweils eine Typbezeichnung zugeordnet sein, welche die Form des jeweiligen Applikators angibt. Beispiele für die Typbezeichnung können sein: "Nadelapplikator", "Kugelkopfapplikator" und dergleichen.

Beispielsweise umfasst die therapierelevante Information eine Größe des Applikators 15. Die Größe des Applikators ist ein wesentliches Kriterium bei der Auswahl eines Applikators in der Brachytherapie, da der Applikator beispielsweise einen Hohlraum im zu bestrahlenden Gewebe möglichst ideal ausfüllen und das Gewebe stützen soll.

Beispielsweise umfasst die therapierelevante Information eine Chargennummer des Applikators 15 und/oder eine Seriennummer des Applikators 15. Die Chargennummer gibt beispielsweise einen Produktionszyklus im Herstellungsverfahren von Applikatoren an. Die Seriennummer ist beispielsweise eine Kennzeichnung für die Herstellungskriterien des Applikators.

Beispielsweise umfasst die therapierelevante Information einen Nutzungszustand des Applikators 15. Der Nutzungszustand kann beispielsweise "neu" oder "gebraucht" oder "verbraucht" sein. Der Nutzungszustand ist "neu", wenn der Applikator noch nicht für eine Bestrahlung verwendet wurde. Der Nutzungszustand kann von der Steuerung auf "gebraucht" oder "verbraucht" geändert werden, wenn der Applikator für eine Bestrahlung verwendet wurde.

Beispielsweise umfasst die therapierelevante Information eine Nutzungsdauer des Applikators 15. Die Nutzungsdauer gibt beispielsweise die zeitliche Dauer oder Häufigkeit an, mit welcher der Applikator für eine Bestrahlung verwendet wurde.

Beispielsweise umfasst die therapierelevante Information eine Bestrahlungsdosis des Applikators 15. Die Bestrahlungsdosis des Applikators 15 gibt die durch vorherige Anwendungen auf den Applikator 15 eingewirkte Gesamtstrahlungsdosis an.

Beispielsweise umfasst die therapierelevante Information ein Ablaufdatum des Applikators 15. Applikatoren werden in einem sterilen Zustand in einer Verpackung bereitgestellt. Die Verpackung gewährleistet den sterilen Zustand jedoch lediglich bis zu dem Ablaufdatum. Daher und aus anderen Gründen darf ein Applikator nur bis zu dem ihm zugewiesenen Ablaufdatum verwendet werden.

Die therapierelevante Information kann einige oder alle der vorangehend genannten Informationen und weitere nicht genannte Informationen umfassen.

In Schritt S104, der auf Schritt S103 folgt, verwendet die Steuerung 27 die empfangene therapierelevante Information bei der Steuerung der Strahlungserzeugungsvorrichtung 3. Mit anderen Worten steuert die Steuerung 27 die Erzeugung von Röntgenstrahlung durch die Strahlungserzeugungsvorrichtung 3 in Abhängigkeit der empfangenen therapierelevanten Information. Beispiele für die Verwendung der therapierelevanten Information durch die Steuerung 27 werden später erläutert.

In einem optionalen Schritt S105, der auf Schritt S104 folgt, wird die therapierelevante Information in dem Datensatz-Datenspeicher aktualisiert. Beispielsweise ändert die Steuerung 27 die therapierelevante Information in dem Datensatz in dem Datensatz-Datenspeicher in Abhängigkeit einer durchgeführten Bestrahlung. Auf diese Weise wird die therapierelevante Information betreffend den zur Durchführung der Bestrahlung verwendeten Applikator aktualisiert, so dass die therapierelevante Information betreffend diesen Applikator auf dem aktuellen Stand ist.

Mit Bezug zu Figur 7 wurde ein Verfahren beschrieben, in welchem die in oder an dem Applikator 15 verkörperte Information eine Identifikationsinformation umfasst. Die Identifikationsinformation dient der (eindeutigen) Identifizierung des zu einer Bestrahlung zu verwenden beabsichtigten Applikators. Basierend auf der Identifikationsinformation wird die therapierelevante Information bezüglich dieses Applikators bezogen. Auf diese Weise ist es ausreichend, dass die in oder an dem Applikator verkörperte Information lediglich eine Identifikationsinformation umfasst. Die in oder an dem Applikator verkörperte Information muss die therapierelevante Information selbst nicht umfassen. Dementsprechend können zur Durchführung dieses Verfahrens einfache Identifikationsvorrichtungen verwendet werden, wie beispielsweise die optischen Kennzeichnungen 31, 33 (siehe Figur 3). Der Datenspeicher 37 muss lediglich eine einzige Information speichern, nämlich die Identifikationsinformation selbst. Daher kann der Datenspeicher 37 einfach und kostengünstig gestaltet sein. Der Datensatz, in welchem die therapierelevante Information betreffend einen Applikator enthalten ist, kann außerhalb des Applikators 15 in dem Datensatz-Datenspeicher gespeichert sein. Der Datensatz-Datenspeicher kann beispielsweise ein Server oder dergleichen sein, welcher einen zentralen Zugriffspunkt für die therapierelevante Information einer Vielzahl von Applikatoren bereitstellt.

Mit Bezug zu Figur 8 wird nun ein Verfahren zur Anwendung des Brachytherapiesystems 1 erläutert, bei welchem die in oder an dem Applikator 15 verkörperte Information die therapierelevante Information selbst umfasst. Dieses Verfahren kann beispielsweise mit dem mit Bezug zu den Figuren 4 bis 6 beschriebenen Brachytherapiesystem 1 durchgeführt werden, bei welchem die therapierelevante Information in dem Datenspeicher 37 enthalten ist, der in dem Applikator 15 angeordnet ist.

In Schritt S201 wird der Applikator 15 auf die Strahlungserzeugungsvorrichtung 3 aufgesetzt. Dieser Schritt entspricht dem Schritt S101 des in Figur 7 gezeigten Verfahrens.

In Schritt S202, der auf Schritt S201 folgt, überträgt das Informationsübertragungssystem 29A, 29B, 29C die in dem Applikator 15 verkörperte Information zu der Steuerung 27, wobei die Information die therapierelevante Information umfasst. Die therapierelevante Information ist in dem Datenspeicher 37 in dem Applikator 15 gespeichert. Im Gegensatz zu dem Verfahren der Figur 7, in welchem die therapierelevante Information von der Steuerung 27 unter Verwendung einer Identifikationsinformation aus einem Datensatz-Datenspeicher außerhalb des Applikators 15 empfangen wurde, wird in dem Verfahren gemäß der Figur 8 die therapierelevante Information in dem Applikator 15 bzw. in dem Datenspeicher 37 in dem Applikator 15 gespeichert und von dort unter Verwendung des Informationsübertragungssystems 29A, 29B, 29C an die Steuerung 27 übertragen.

In Schritt S203, der auf Schritt S202 folgt, verwendet die Steuerung 27 die empfangene therapierelevante Information zur Steuerung der Strahlungserzeugungsvorrichtung 3. Schritt S203 entspricht Schritt S103 der Figur 7.

In einem optionalen Schritt S204, der auf Schritt S203 folgt, wird die therapierelevante Information in dem Datenspeicher 37 des Applikators 15 aktualisiert. Beispielsweise weist die Steuerung 27 das Informationsübertragungssystem 29 an, die therapierelevante Information in dem Datenspeicher 27 in Abhängigkeit einer durchgeführten Bestrahlung zu ändern.

Beispielsweise fügt die Steuerung 27 zu der therapierelevanten Information in dem Datenspeicher 27 eine Information betreffend eine Verwendung des Applikators (15) bei der Bestrahlung und/oder eine Bestrahlungsdauer des Applikators (15) bei der Bestrahlung und/oder eine auf den Applikator (15) angewendete Dosis hinzu und/oder aktualisiert eine entsprechende Information in der therapierelevanten Information in dem Datenspeicher 27.

Nachfolgend werden einige Beispiele für die Verwendung der therapierelevanten Information bei der Steuerung der Strahlungserzeugungsvorrichtung 3 gemäß den Schritten S104 und S204 erläutert.

Beispielsweise vergleicht die Steuerung die therapierelevante Information mit einem Bestrahlungsplan, der die durchzuführende Bestrahlung definiert. Basierend auf dem Ergebnis des Vergleichs steuert die Steuerung die Erzeugung von Röntgenstrahlung durch die Strahlungserzeugungsvorrichtung 3 und gibt gegebenenfalls Warnungen aus.

Ein Bestrahlungsplan kann eine vorgeschriebene Typbezeichnung definieren. Die Steuerung 27 kann die in dem Bestrahlungsplan angegebene vorgeschriebene Typbezeichnung mit der Typbezeichnung in der therapierelevanten Information vergleichen und die Erzeugung von Röntgenstrahlung verhindern, wenn der Vergleich ergibt, dass die Typbezeichnung des auf die Strahlungserzeugungsvorrichtung 3 aufgesetzten Applikators 15 nicht der vorgeschriebenen Typbezeichnung entspricht.

Ein Bestrahlungsplan kann eine vorgeschriebene Größe des zu verwendenden Applikators definieren. Die Steuerung 27 kann die in dem Bestrahlungsplan angegebene vorgeschriebene Größe mit der Größe des Applikators in der therapierelevanten Information vergleichen und die Erzeugung von Röntgenstrahlung verhindern, wenn der Vergleich ergibt, dass die Größe des auf die Strahlungserzeugungsvorrichtung 3 aufgesetzten Applikators 15 nicht der vorgeschriebenen Größe entspricht.

Ein Bestrahlungsplan kann eine vorgeschriebene Chargennummer und/oder eine vorgeschriebene Seriennummer des zu verwendenden Applikators definieren. Die Steuerung 27 kann die in dem Bestrahlungsplan angegebene vorgeschriebene Chargennummer/Seriennummer mit der Chargennummer/Seriennummer des Applikators in der therapierelevanten Information vergleichen und die Erzeugung von Röntgenstrahlung verhindern, wenn der Vergleich ergibt, dass die Chargennummer/Seriennummer des auf die Strahlungserzeugungsvorrichtung 3 aufgesetzten Applikators 15 nicht der vorgeschriebenen Chargennummer/Seriennummer entspricht.

Ein Bestrahlungsplan kann einen vorgeschriebenen Nutzungszustand definieren. Die Steuerung 27 kann den in dem Bestrahlungsplan angegebenen vorgeschriebenen Nutzungszustand mit dem Nutzungszustand in der therapierelevanten Information vergleichen und die Erzeugung von Röntgenstrahlung verhindern, wenn der Vergleich ergibt, dass der Nutzungszustand des auf die Strahlungserzeugungsvorrichtung 3 aufgesetzten Applikators 15 nicht dem vorgeschriebenen Nutzungszustand entspricht.

Ein Bestrahlungsplan kann eine zulässige Nutzungsdauer definieren. Die Steuerung 27 kann die in dem Bestrahlungsplan angegebene zulässige Nutzungsdauer mit der Nutzungsdauer in der therapierelevanten Information vergleichen und die Erzeugung von Röntgenstrahlung verhindern, wenn der Vergleich ergibt, dass die Nutzungsdauer des auf die Strahlungserzeugungsvorrichtung 3 aufgesetzten Applikators 15 die zulässige Nutzungsdauer überschreitet.

Ein Bestrahlungsplan kann eine zulässige Bestrahlungsdosis definieren. Die Steuerung 27 kann die in dem Bestrahlungsplan angegebene zulässige Bestrahlungsdosis mit der Bestrahlungsdosis in der therapierelevanten Information vergleichen und die Erzeugung von Röntgenstrahlung verhindern, wenn der Vergleich ergibt, dass die Bestrahlungsdosis des auf die Strahlungserzeugungsvorrichtung 3 aufgesetzten Applikators 15 die zulässige Bestrahlungsdosis überschreitet.

Beispielsweise kann die Steuerung 27 dazu konfiguriert sein, die Erzeugung von Röntgenstrahlung zu verhindern, wenn die Steuerung feststellt, dass ein in der therapierelevanten Information angegebenes Ablaufdatum des Applikators verstrichen ist. In dieser Ausführungsform prüft die Steuerung das Ablaufdatum und verhindert die Erzeugung von Röntgenstrahlung, wenn das Ablaufdatum verstrichen ist. Auf diese Weise wird sichergestellt, dass eine Bestrahlung nur mit einem ausreichend sicheren Applikator durchgeführt werden kann.

Neben oder alternativ zu dem Verhindern der Erzeugung von Röntgenstrahlung kann die Steuerung in Abhängigkeit der therapierelevanten Information Warnungen ausgeben, die einen Bediener des Brachytherapiesystems darauf hinweisen, dass der aktuell auf die Strahlungserzeugungsvorrichtung 3 aufgesetzte Applikator dem Bestrahlungsplan nicht entspricht.

Das vorangehend beschriebene Brachytherapiesystem 1 erhöht die Sicherheit bei der Durchführung von therapeutischen Bestrahlungen. So kann sichergestellt werden, dass der zum Einsatz kommende Applikator einem Bestrahlungsplan entspricht. Fehler durch menschliches Versagen können hierbei ausgeschlossen werden, da der aufgesetzte Applikator entweder durch eine Identifikationsinformation eindeutig identifiziert werden kann oder die therapierelevante Information betreffend den Applikator in dem Applikator selbst gespeichert ist und an die Steuerung übertragen wird, wenn der Applikator auf die Strahlungserzeugungsvorrichtung aufgesetzt ist.

## Patentansprüche

1. Brachytherapiesystem (1), umfassend:
eine Strahlungserzeugungsvorrichtung (3), welche dazu konfiguriert ist, Röntgenstrahlung zu erzeugen;
eine Steuerung (27) zum Steuern der Strahlungserzeugungsvorrichtung (3);
einen Applikator (15), welcher so ausgebildet ist, dass er auf die Strahlungserzeugungsvorrichtung (3) aufgesetzt werden kann;
ein Informationsübertragungssystem (29, 29A, 29B, 29C), welches dazu konfiguriert ist, eine in oder an dem Applikator (15) verkörperte Information zu erfassen, wenn der Applikator (15) auf die Strahlungserzeugungsvorrichtung (3) aufgesetzt ist, und an die Steuerung (27) zu übertragen;
wobei die Steuerung (27) dazu konfiguriert ist, die Erzeugung von Röntgenstrahlung durch die Strahlungserzeugungsvorrichtung (3) in Abhängigkeit der erfassten Information zu steuern.

2. Brachytherapiesystem nach Anspruch 1,
wobei die Information durch eine optische Kennzeichnung (31, 33) realisiert ist und das Informationsübertragungssystem (29) ein Lesegerät (35) umfasst,
welches dazu konfiguriert ist, die optische Kennzeichnung (31, 33) an dem Applikator (15) zu lesen, wenn der Applikator (15) auf die Strahlungserzeugungsvorrichtung (3) aufgesetzt ist.

3. Brachytherapiesystem nach Anspruch 1,
wobei die Information durch ein Signal in einem Datenspeicher (37) realisiert ist, wobei der Datenspeicher (37) in dem Applikator (15) angeordnet ist, und wobei das Informationsübertragungssystem (29A, 29B, 29C) eine Empfangseinheit (39, 45, 53) umfasst, welche dazu konfiguriert ist, die Information aus dem Datenspeicher (37) zu empfangen, wenn der Applikator (15) auf die Strahlungserzeugungsvorrichtung (3) aufgesetzt ist.

4. Brachytherapiesystem nach Anspruch 3,
wobei der Applikator (15) an seiner Oberfläche (18) wenigstens einen elektrischen Kontakt (41) zum Herstellen einer drahtgebundenen Kommunikationsverbindung zwischen dem Datenspeicher (37) und der Empfangseinheit (39) umfasst.

5. Brachytherapiesystem nach Anspruch 3,
wobei das Informationsübertragungssystem (29B) ein in oder an dem Applikator (15) angeordnetes Kommunikationsmodul (47) umfasst, welches dazu konfiguriert ist, die Information drahtlos an die Empfangseinheit (45) zu übertragen.

6. Brachytherapiesystem nach Anspruch 3,
wobei das Informationsübertragungssystem (29C) ein optisches Informationsübertragungssystem umfasst, welches ein Lichtsignal (51) zur Übertragung der Information verwendet.

7. Brachytherapiesystem nach einem der Ansprüche 1 bis 6,
wobei die Information eine Identifikationsinformation umfasst, welche den Applikator (15) identifiziert;
wobei die Steuerung (27) ferner dazu konfiguriert ist, einen der Identifikationsinformation zugeordneten Datensatz aus einem Datensatz-Datenspeicher zu empfangen, wobei der Datensatz therapierelevante Information enthält; und
wobei die Steuerung (27) dazu konfiguriert ist, die Erzeugung von Röntgenstrahlung durch die Strahlungserzeugungsvorrichtung (3) in Abhängigkeit der empfangenen therapierelevanten Information zu steuern.

8. Brachytherapiesystem nach Anspruch 7,
wobei die Steuerung (27) ferner dazu konfiguriert ist, die therapierelevante Information in dem Datensatz in dem Datensatz-Datenspeicher in Abhängigkeit einer durchgeführten Bestrahlung zu ändern.

9. Brachytherapiesystem nach einem der Ansprüche 3 bis 6,
wobei die Information therapierelevante Information umfasst; und
wobei die Steuerung (27) dazu konfiguriert ist, die Erzeugung von Röntgenstrahlung durch die Strahlungserzeugungsvorrichtung (3) in Abhängigkeit der empfangenen therapierelevanten Information zu steuern.

10. Brachytherapiesystem nach Anspruch 9,
wobei die Steuerung (27) ferner dazu konfiguriert ist, die therapierelevante Information in dem Datenspeicher (37) in dem Applikator (15) in Abhängigkeit einer durchgeführten Bestrahlung zu ändern.

11. Brachytherapiesystem nach Anspruch 8 oder 10,
wobei die Steuerung (27) ferner dazu konfiguriert ist, Information betreffend eine Verwendung des Applikators (15) bei der Bestrahlung, eine Bestrahlungsdauer des Applikators (15) bei der Bestrahlung und/oder eine bei der Bestrahlung auf den Applikator (15) angewendete Dosis zu der therapierelevanten Information hinzuzufügen und/oder in der therapierelevanten Information zu aktualisieren.

12. Brachytherapiesystem nach einem der Ansprüche 7 bis 11,
wobei die therapierelevante Information umfasst: eine Typbezeichnung des Applikators (15) und/oder eine Größe des Applikators (15) und/oder eine Chargennummer des Applikators (15) und/oder eine Seriennummer des Applikators (15) und/oder einen Nutzungszustand des Applikators (15) und/oder eine Nutzungsdauer des Applikators (15) und/oder eine Bestrahlungsdosis des Applikators (15) und/oder ein Ablaufdatum des Applikators (15).

13. Brachytherapiesystem nach einem der Ansprüche 7 bis 12,
wobei die Steuerung (27) dazu konfiguriert ist, die therapierelevante Information mit einem Bestrahlungsplan zu vergleichen und basierend auf dem Ergebnis des Vergleichs die Erzeugung von Röntgenstrahlung und die Ausgabe von Warnungen zu steuern.
